(19) Europäisches Patentamt
European Patent Office
Office européen des brevets

(11) Veröffentlichungsnummer: **0 015 584**
**A2**

# (12) EUROPÄISCHE PATENTANMELDUNG

(21) Anmeldenummer: **80101241.0**

(22) Anmeldetag: **11.03.80**

(51) Int. Cl.³: **C 07 H 19/10**, C 07 H 19/20, C 07 H 19/04, C 07 F 9/65, C 07 F 9/58, A 61 K 31/70, A 61 K 31/675

(30) Priorität: **12.03.79 DE 2909709**
**28.05.79 DE 2921623**

(43) Veröffentlichungstag der Anmeldung: **17.09.80**
**Patentblatt 80/19**

(84) Benannte Vertragsstaaten: **CH DE FR GB IT SE**

(71) Anmelder: **Gauri, Kailash Kumar, Dr., D-2359 Lentföhrden (DE)**

(72) Erfinder: **Gauri, Kailash Kumar, Dr., D-2359 Lentföhrden (DE)**

(74) Vertreter: **Kinzebach, Werner, Dr. Patentanwälte et al, Reitstötter J. Prof.Dr.Dr. Kinzebach W. Dr. & Partner Bauerstrasse 22 Postfach 780, D-8000 München 43 (DE)**

(54) **Neue Nukleotide, Verfahren zu ihrer Herstellung und Arzneimittel.**

(57) Nucleotide der allgemeinen Formel I:

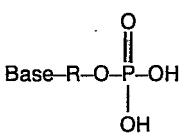

(I)

worin «Base» einen Purinbasenrest, Pyrimidinbasenrest, Pyridon-2-basenrest oder 3,6-Dihydropyridon-2-basenrest bedeutet, der in verschiedener Weise substituiert sein kann und worin

R einen Pentofuranosylrest der allgemeinen Formel darstellt:

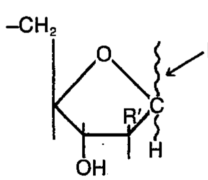


worin R' für ein Halogenatom, H oder OH steht; oder

R eine gerade oder verzweigte Alkylenkette mit 2 bis 6 Kohlenstoffatomen, die gegebenenfalls ein Äther-O-Atom in der Kette enthält, bedeutet,

wobei Halogen für F, Cl, Br, J steht;
sowie die Cyclophosphatester mit der OH-Gruppe in 3-Stellung des Pentofuranosylrests R und ein Verfahren zu ihrer Herstellung. Die Verbindungen besitzen eine antivirale Wirksamkeit, insbesondere gegenüber resistenten Herpes Viren.

EP 0 015 584 A2

Die Erfindung betrifft neue Nukleotide mit antiviraler Wirksamkeit und das Zellwachstum induzierender und fördernder Wirkung, Verfahren zur Herstellung dieser Verbindungen und Arzneimittel, die diese Verbindungen enthalten.

Einige Nukleosid-Analoga sind als potentielle antiherpetische Substanzen bereits bekannt. Hierzu gehören unter anderem Joddesoxyuridin, Äthyldesoxyuridin, Trifluorthymidin, Arabinofuranosyladenin sowie das Acycloguanosin. Auch dem natürlichen Desoxythymidin kommt eine gewisse Wirksamkeit gegenüber Herpes simplex-Viren (HS-Viren) zu. Obwohl diese Substanzen bei der primären Anwendung mit Erfolg zur Bekämpfung von Herpes Infektionen therapeutisch eingesetzt werden können, haben sie den Nachteil, daß die Herpesviren sehr bald Resistenz gegenüber diesen Verbindungen entwickeln, so daß eine sekundäre Anwendung an dieser Resistenz scheitert.

Überraschenderweise wurde nun gefunden, daß die erfindungsgemäßen Monophosphate derartiger Nukleoside auch gegenüber den resistenten Virenstämmen sehr wirksam sind. Diese Wirksamkeit der Nukleosidphosphate gegenüber den Herpesviren ist in mehrfacher Hinsicht überraschend. Beispielsweise ist es allgemein bekannt, daß im Gegensatz zu den Nukleosiden die Phosphate nicht in der Lage sind, die Zellmembran zu durchdringen, somit war für die Phosphate eine Wirksamkeit nicht zu erwarten. Darüberhinaus enthalten die Zellmembranen Enzyme, die die Nukleosidphosphate spalten und daher diese Verbindungen inaktivieren. Folglich war auch bei unterstellter Transportfähigkeit aus dem vorgenannten Grunde von den Phosphaten keine Wirksamkeit zu erwarten.

Überraschenderweise wurde gefunden, daß die erfindungsgemäßen Nucleosidphosphate als solche bei Infektionen durch HS-Viren therapeutisch wirsam sind und daß deren Wirksamkeit sich auch auf resistente HS-Virenstämme erstreckt.

Gegenstand der vorliegenden Erfindung sind neue Nucleotide der allgemeinen Formel I:

$$\text{Base} - \text{R} - \text{O} - \overset{\displaystyle \text{O}}{\overset{\|}{\underset{\underset{\displaystyle \text{OH}}{|}}{\text{P}}}} - \text{OH} \qquad \text{(I)}$$

worin:

"Base" einen Purinbasenrest, Pyrimidinbasenrest, Pyridon-2-basen-rest oder 3,6-Dihydropyridon-2-basenrest bedeutet, wobei

(a) der Pyrimidinbasenrest und der Pyridon-2-basenrest in 4-Stellung eine Aminogruppe tragen können;

(b) der Pyridon-2-basenrest in 5-Stellung durch ein Halogen substituiert sein kann; und

(c) der Pyrimidinbasenrest in 5-Stellung durch Halogen, einen geradkettigen oder verzweigten $(C_2-C_6)$Alkylrest, $(C_2-C_6)$Alkenylrest, $(C_2-C_6)$Halogenalkylrest oder $(C_2-C_6)$Halogenalkenylrest substituiert sein kann, in 6-Stellung durch Chlor substituiert sein kann und, wenn in 5-Stellung ein $(C_2-C_6)$Alkylrest steht, in 3-Stellung durch einen geradkettigen oder verzweigten $(C_1-C_4)$Alkylrest substituiert sein kann; und

R einen Pentofuranosylrest der allgemeinen Formel II darstellt:

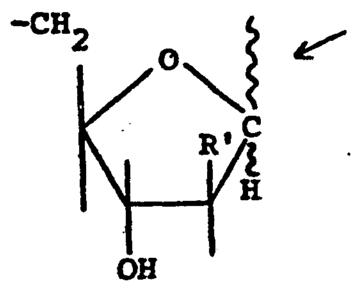

Bindung zur Base

(II)

worin R' für ein Halogenatom, H oder OH steht; oder

R eine gerade oder verzweigte Alkylenkette mit 2 bis 6 Kohlenstoffatomen, die gegebenenfalls ein Äther-O-Atom in der Kette enthält, bedeutet,

wobei Halogen für F, Cl, Br, J steht;

sowie die Cyclophosphatester mit der OH-Gruppe in 3-Stellung des Pentofuranosylrests R.

Nach einer bevorzugten Ausführungsform schafft die Erfindung Nucleotide der vorstehenden Art, die dadurch gekennzeichnet sind, daß der Rest "Base" einen Purinbasenrest, Pyrimidinbasenrest oder Pyridon-2-basenrest bedeutet, wobei

(a) der Pyrimidinbasenrest und der Pyridon-2-basenrest in 4-Stellung eine Aminogruppe tragen können,

(b) der Pyridon-2-basenrest in 5-Stellung durch ein Fluor-, Chlor-, Brom- oder Jodatom substituiert sein kann; und

(c) der Pyrimidinbasenrest in 5-Stellung durch Halogen, einen geradkettigen oder verzweigten $(C_2-C_6)$Alkylrest, $(C_2-C_6)$Alkenylrest, $(C_2-C_6)$Halogenalkylrest oder $(C_2-C_6)$Halogenalkenylrest substituiert sein kann, wobei Halogen für Fluor, Chlor, Brom oder Jod steht; und

R einen Pentofuranosylrest der Formel II:

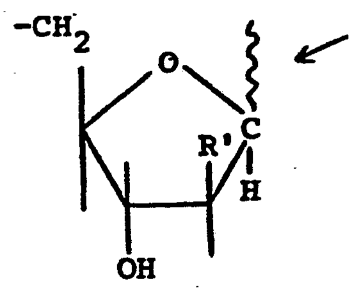

Bindung zur Base

(II)

worin R' für ein Fluor-, Chlor-, Brom- oder Jodatom steht; oder

eine gerade oder verzweigte Alkylenkette mit 2 bis 6 C-Atomen, die gegebenenfalls ein Äther-O-Atom in der Kette enhält, bedeutet.

Nach einer weiteren bevorzugten Ausführungsform sind die Nucleotide dadurch gekennzeichnet, daß die Purinbase das Purin selbst oder Adenin, Guanin, Hypoxanthin oder Xanthin ist und die Pyrimidinbase das Pyrimidin selbst oder Cytosin, Uracil, 5-Methylcytosin oder 5-Hydroxymethylcytosin ist.

Bevorzugt sind insbesondere diejenigen Nucleotide, bei denen der Substituent in 5-Stellung der Pyrimidinbase ein Äthyl-, Propyl-, Butyl-, Pentyl-, Vinyl-, Allyl-, 2-Halovinyl- oder 3-Haloalkylrest, wobei Halo für F, Cl und Br steht, ist.

Ganz besonders bevorzugte Ausführungsformen der Erfindung sind diejenigen Nucleotide, bei denen die Pyrimidinbase Uracil ist und R für den Pentofuranosylrest steht.

Nach einer ebenfalls bevorzugten Ausführungsform werden Nucleotide geschaffen, in denen der Begriff "Base" für einen Uracilrest steht, und die der allgemeinen Formel III:

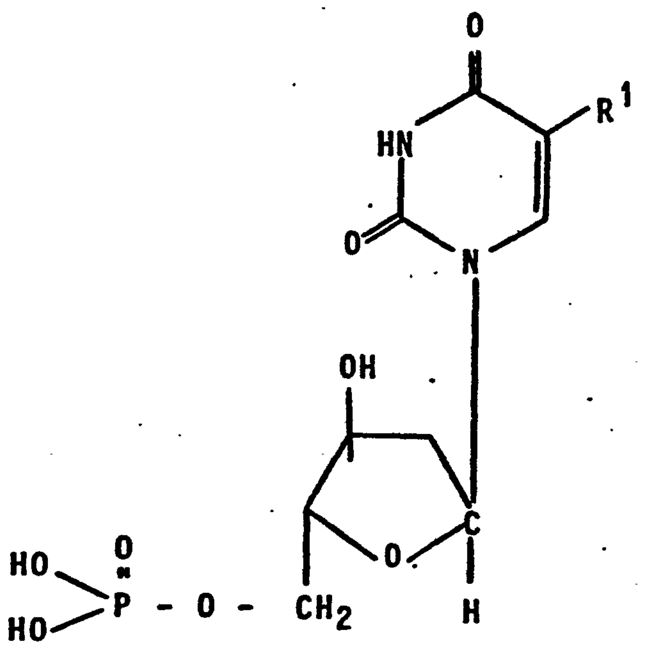

(III)

worin $R^1$ einen verzweigten oder geradkettigen $(C_2-C_6)$Alkylrest darstellt;

sowie der allgemeinen Formel IV:

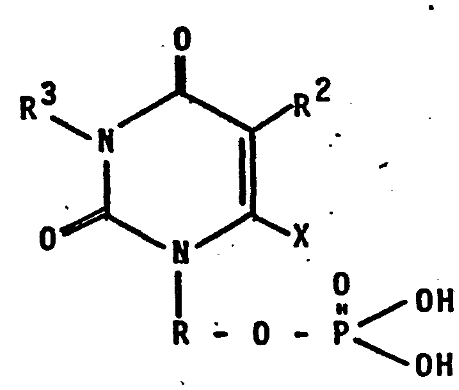

(IV)

worin

$R^2$ für einen geradkettigen oder verzweigten $(C_2-C_6)$Alkylrest steht und

$R^3$ für ein Wasserstoffatom oder einen geradkettigen oder verzweigten $(C_1-C_4)$Alkylrest steht,

X ein Wasserstoff- oder Chloratom bedeutet; und R die in Anspruch 1 genannten Bedeutungen hat.

Die erfindungsgemäßen Verbindungen werden hergestellt, indem man eine Verbindung der allgemeinen Formel V:

$$\text{Base} - R - OH \qquad \text{(V)}$$

worin die Reste Base und R die oben genannten Bedeutungen haben,
entweder mit Phosphoroxychlorid in einem Lösungsmittel phosphoryliert und das erhaltene Phosphat der Formel I mit Äther, Petroläther oder einem Gemisch dieser Lösungsmittel aus dem Reaktionsgemisch ausfällt oder

ein Base-Metallsalz, insbesondere ein Base-Alkali- oder Quecksilbersalz, mit einer Verbindung der allgemeinen Formel (VI):

$$\begin{array}{l} \qquad\qquad\quad O \\ \qquad\qquad\quad \| \\ H_5C_6 - O - P - O - R - Cl \\ \qquad\qquad\quad | \\ \quad H_5C_6 - O \end{array} \qquad \text{(VI)}$$

wobei "Base" und R die oben genannten Bedeutungen haben,

umsetzt und anschließend die Phenylgruppe am Phosphatrest durch Behandlung mit Phosphordiesterase entfernt.

Vorteilhafte Lösungsmittel bei der Phosphorylierung sind Trimethyl- bzw. Triäthylphosphat, wobei man aber auch von einer Lösung des nicht phosphorylierten Ausgangsproduktes der Formel V in einem niedrigen Alkohol mit 1 bis 4 C-Atomen ausgehen kann und dieser Lösung ein Gemisch von Phosphoroxychlorid und Triäthyl- bzw. Trimethylphosphat zusetzt, das soviel Phosphoroxychlorid enthält, daß einerseits der in die Reaktion eingebrachte Lösungsmittel-Alkohol verestert wird und andererseits das in die Reaktion eingesetzte Ausgangsprodukt der Formel V verestert wird. Ein etwa stöchiometrischer Anteil Phosphoroxychlorid ist im allgemeinen für die Phosphorylierung der Purinverbindungen ausreichend, während bei den Pyrimidinverbindungen ein Überschuß an Phosphoroxychlorid erforderlich ist.

Zur Herstellung der erfindungsgemäßen Verbindungen, bei denen R für eine gerade oder verzweigte Alkylenkette mit 2 bis 6 C-Atomen steht, die gegebenenfalls ein Äther-O-Atom in der Kette enthalten kann, geht man vorteilhafterweise von dem Mono-Quecksilbersalz des Pyrimidins bzw. dem Kaliumsalz des Pyridons-2 aus und setzt dieses Salz mit einer Verbindung der allgemeinen Formel VI:

$$H_5C_6-O-\underset{\underset{O}{\|}}{\overset{\overset{O-C_6H_5}{|}}{P}}-O-R-Cl \qquad (VI)$$

um und entfernt anschließend die Phenylreste am Phosphatrest durch Behandlung mit Phosphordiesterase.

Der im vorliegenden Zusammenhang verwendete Begriff "Purinbase" stellt eine Bezeichnung für eine Gruppe wichtiger, in der Natur weit verbreiteter und an menschlichen und tierischen Stoffwechselvorgängen beteiligter Verbindungen, die sich vom Grundkörper Purin durch Substitution mit OH, $NH_2$, in 2-, 6- und 8-Stellung und/oder mit $CH_3$ in 1-, 3-, 7-Stellung ableiten, dar.

Geeignete Purinbasen sind z.B. Purin, Adenin, Guanin, Pypoxanthin und Xanthin.

In gleicher Weise werden im vorliegenden Zusammenhang unter Pyrimidinbasen solche Verbindungen verstanden, die sich vom Pyrimidin ableiten, insbesondere in Form von Hydroxy- und Aminoderivaten.

Beispiele für geeignete Pyrimidinbasen sind Pyrimidin, Cytosin, Uracil, 5-Methylcytosin, 5-Hydroxymethylcytosin und Pseudouridin. Die mit der Base verknüpften Pentofuranosylreste R können in der α- oder ß-Form vorliegen. Je nach Art der Verknüpfung spricht man in diesem Zusammenhang von α- oder ß-Anomeren. Die Erfindung umfaßt sowohl die α-Anomeren, wie auch die ß-Anomeren, sowie sämtliche Gemische davon. Das soll anmeldungsgemäß durch Verwendung wellenförmiger Bindungsstriche zwischen dem Pentofuranosylrest R und der Base in den allgemeinen Formeln zum Ausdruck gebracht werden.

Die im vorliegenden Zusammenhang verwendeten Begriffe "$(C_2-C_6)$Alkylreste" sollen geradkettige oder verzweigte Alkylreste umfassen, beispielsweise Äthyl, n-Propyl, Isopropyl, n-Butyl, sek.-Butyl. Isobutyl, tert.-Butyl, n-Pentyl, Isopentyl, n-Hexyl, Isohexyl, und dergleichen. Diese Definition gilt auch für den Begriff $(C_2-C_6)$Halogenalkylreste.

Wenn im vorliegenden Zusammenhang der Begriff "$(C_2-C_6)$Alkenylrest" verwendet wird, so soll dieser Ausdruck geradkettige oder verzweigte Alkenylreste umfassen, beispielsweise Äthylen, Propylen-1, Propylen-2, n-Butylen, Isobutylen, tert.-Butylen, n-Pentylen, Isopentylen, n-Hexylen, Isohexylen, und dergleichen. Dasselbe gilt für $(C_2-C_6)$Halogenalkenylreste.

Die erfindungsgemäßen Verbindungen können vorteilhaft auch als Cyclophosphatester vorliegen, bei denen eine OH-Gruppe des Phosphatesters mit der OH-Gruppe in 3-Stellung des Pentofuranosylrestes R verestert ist, wie nachstehend dargestellt:

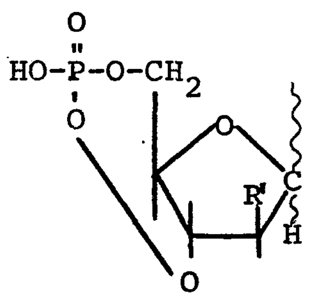

Die Ausgangsprodukte, insbesondere die Ausgangsnucleoside (Base-Pentofuranosyl-Verbindung) sind bekannt oder werden nach bekannten Verfahren hergestellt, z.B. gemäß DE-OS 1 620 185.

Beispielhafte Ausführungsformen der erfindungsgemäßen Verbindungen sind folgende:

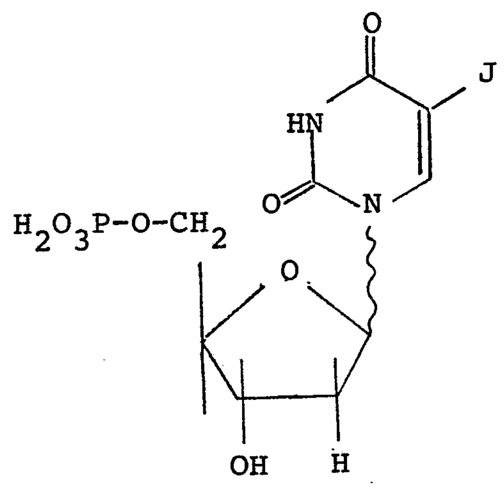

(F; Cl; Br)

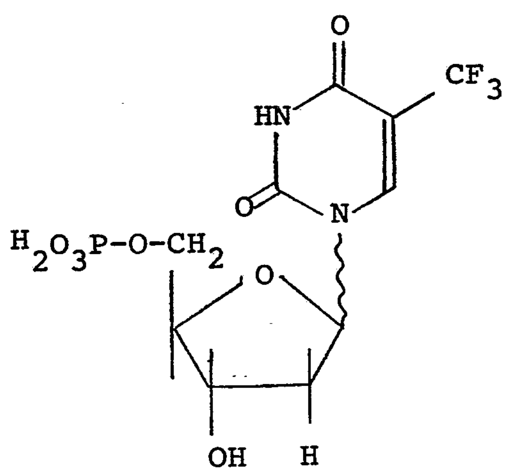

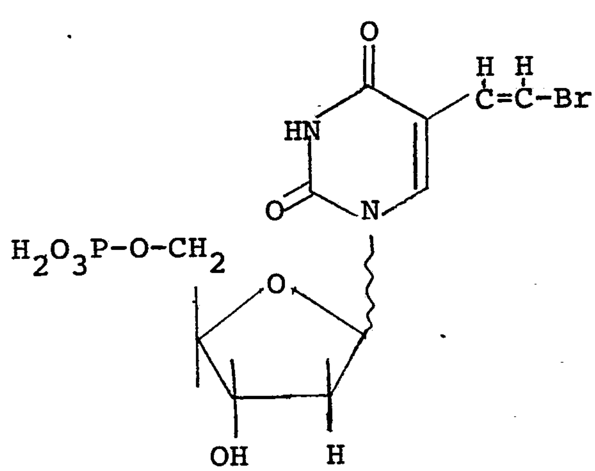

$(CH_2-CH=CH_2)$

$(CH_2-CH=CH-F)$

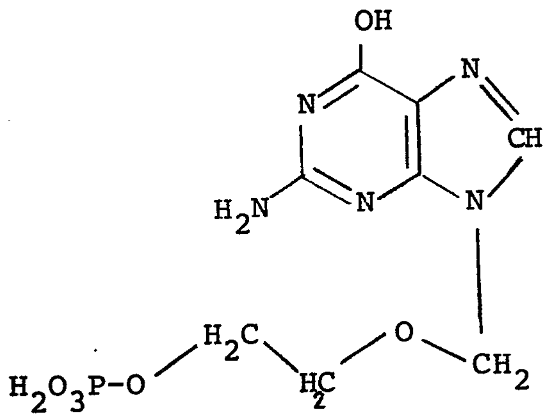

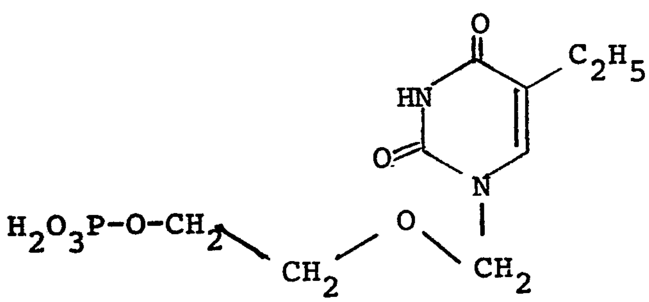

$(-C_3H_7; -C_4H_9)$

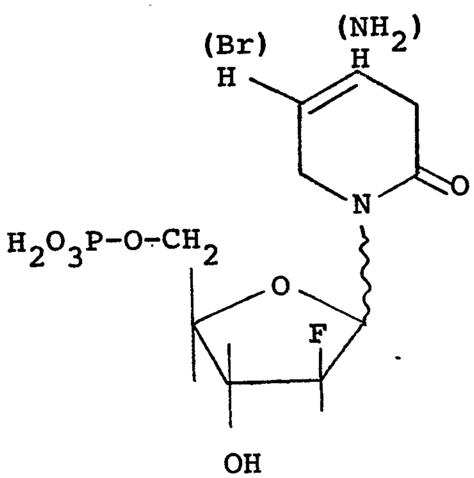

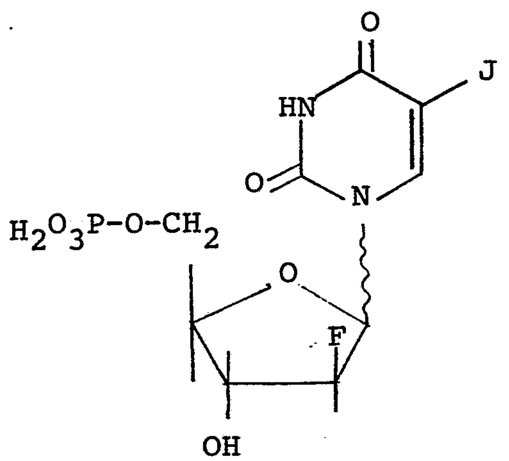

(Cl; Br; F)

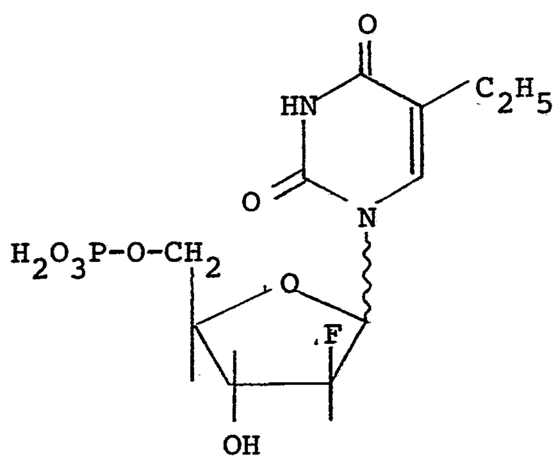

($-C_3H_7$; $-C_4H_9$; $-C_6H_{11}$)

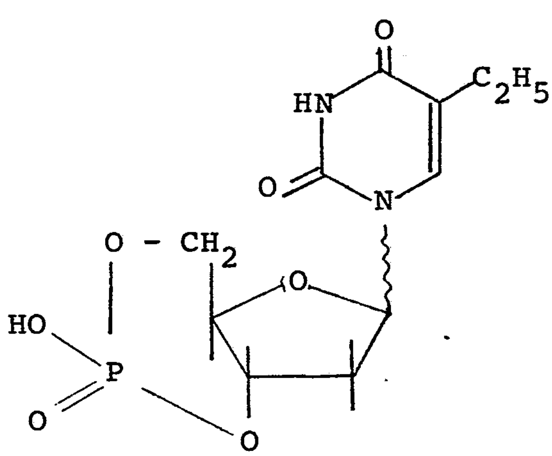

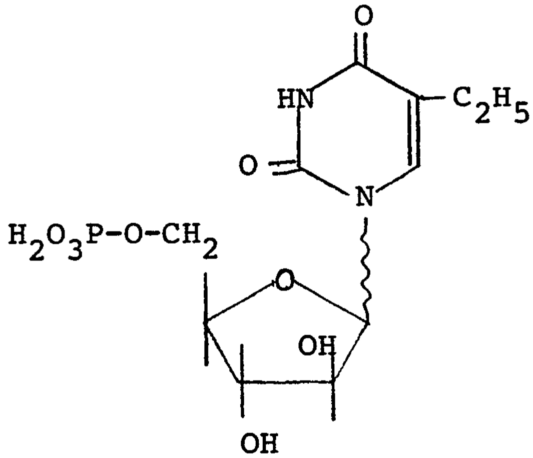

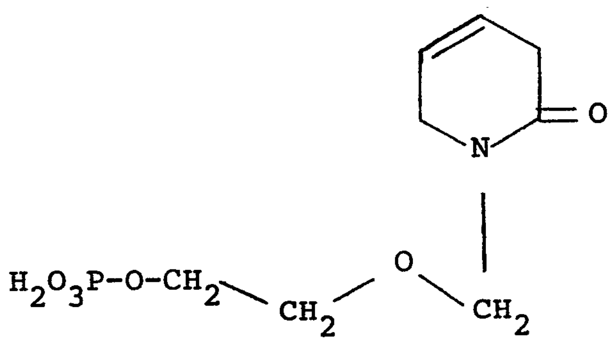

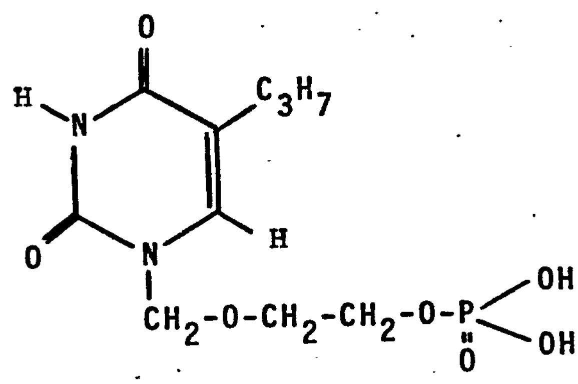

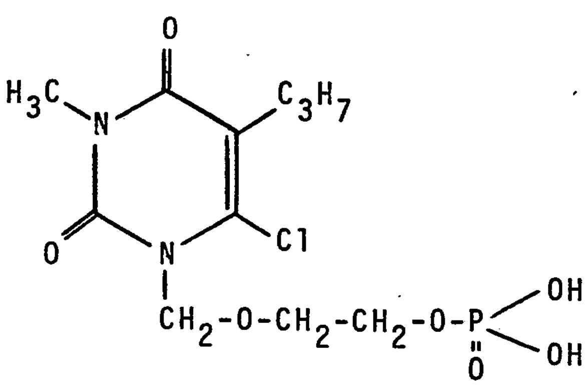

Die Verbindungen der Formel III sind α-5-Niedrigalkyl-2'-deoxyuridin-5'-monophosphate, die entsprechenden β-Formen können in analoger Weise hergestellt werden. Bevorzugt sind 5-Äthyl- bzw. 5-Propyl-2'-deoxyuridin-5'-monophosphat in der α- und/oder β-Form.

Die neuen erfindungsgemäßen Verbindungen haben überraschende pharmakologische Eigenschaften, sie wirken insbesondere virostatisch und sie haben eine das Zellwachstum induzierende und fördernde Wirkung.

Die folgenden Beispiele dienen zur weiteren Erläuterung der Erfindung, ohne sie einzuschränken.

## Beispiel 1

α-5-Äthyl-2'-deoxyuridin-5'-monophosphat

α-5-Äthyl-2'-deoxyuridin (α-ÄDU) 25,6 g (0,1 Mol) wird mit 5 ml Äthanol angefeuchtet und bei ca. 0°C mit einem Gemisch aus 120 ml $POCl_3$ und 100 ml Triäthylphosphat versetzt. Nach etwa 1-stündigem Rühren bei Zimmertemperatur erhält man eine klare Lösung. Bei der DC (Dünnschichtchromatographie)-Kontrolle zeigt sich, daß die Phosphorylierung insgesamt nach etwa 2 Stunden fast abgeschlossen ist. Zur Beendigung der Reaktion wird die klare Lösung mit 200 ml Äther verdünnt und anschließend solange mit Petroläther unter Rühren versetzt bis keine weitere Trübung mehr auftritt. Nach Abkühlung auf -20°C wird der klare Überstand abdekantiert und der flockige Rückstand mehrmals mit Äther/Petroläther-Gemisch 1:1 gründlich nachgewaschen. Zur Entfernung der Lösungsmittelreste empfiehlt es sich, den Rückstand einige Zeit an ein Ölpumpenvakuum anzuschließen. Danach

wird der Rückstand in Natriumbicarbonat-Lösung aufgenommen und anschließend schonend im Vakuum bis zur Trockne eingedampft.

Das farblose Produkt besteht bis zu 70 % aus dem α-5-Äthyl-2'-deoxyuridin-5'-monophosphat; es enthält als Verunreinigung neben dem Ausgangsprodukt α-ÄDU noch etwas α-ÄDU-3',5'-diphosphat. Für pharmazeutische und pharmakologische Zwecke ist das Reaktionsprodukt genügend rein.

## Beispiel 2

ß-D-Arabinofuranosyladenin-5'-monophosphat

2,6 g ß-D-Arabinofuranosyladenin (ca. 0,01 Mol) werden bei ca. 0°C mit einem Gemisch aus 2 ml $POCl_3$ und 20 ml Triäthylphosphat versetzt und bei ca. +10°C solange gerührt bis das Ausgangsprodukt fast vollständig umgesetzt ist (ca. 45 Min. ermittelt durch DC-Kontrolle). Die klare Lösung wird dann solange mit Äther verdünnt bis keine weitere Trübung mehr auftritt. Nach kurzem Stehen im Kühlschrank wird das Lösungsmittel abdekantiert, der Niederschlag mehrmals mit Äther gewaschen, in möglichst wenig Äthanol gelöst und nochmals mit Äther gefällt. Nach Abkühlung auf -20°C wird der Oberstand abdekantiert. Der erhaltene Rückstand (ca. 2,5 g) besteht zu über 90 % aus dem gewünschten ß-D-Arabinofuranosyladenin-5'-monophosphat.

Auf die gleiche Weise läßt sich ausgehend von der α-Verbindung das α-D-Arabinofuranosyladenin-5'-monophosphat herstellen.

## B e i s p i e l 3

**3,5-Diäthyluracil-1-propyl-3'-phosphat**

3,5-Diäthyl-1-(3'-hydroxypropyl)-uracil (2,2 g, ca. 0,01 Mol) wird bei ca. 0°C mit einem Gemisch von 2 ml $POCl_3$ und 20 ml Triäthylphosphat versetzt und nach dem Verfahren von Beispiel 2 aufgearbeitet. Anstelle von Äther wird Petroläther verwendet. Die Ausbeute an gewünschtem Phosphatderivat beträgt 80 % (ermittelt durch DC). Die Ausbeute an Rohprodukt beträgt 1,8 g.

Auf die gleiche Weise läßt sich auch das 3-Methyl-5-propyl-6-chlor-uracil-1-(2'-oxabutyl-4'-phosphat) der Formel

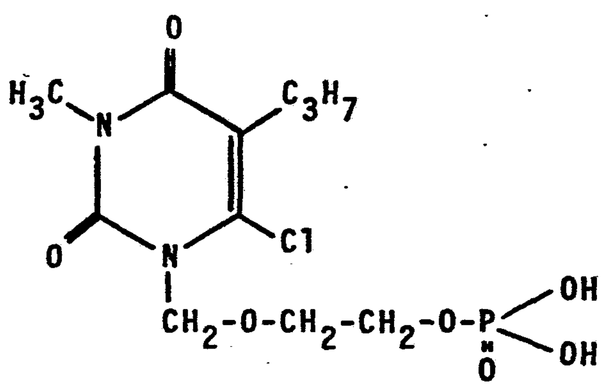

aus dem entsprechenden 3-Methyl-5-propyl-6-chloruracil-1-(2'-oxabutan-4'-ol) herstellen.

Dem α-ÄDU-Monophosphat kommt eine ungewöhnliche Eigenschaft zu, die vom Fachmann nicht vorhersehbar war. Es ist nämlich in der Lage, die Vermehrung von Herpes-Simplex-Viren zu hemmen. In Gewebekulturen von Hühnerembryofibroblasten hemmt α-ÄDU-MP in

einer Konzentration von 10 µg/ml deutlich die Virusvermehrung. Unter gleicher Bedingung ist α-ÄDU selbst ohne Wirkung.

Das erfindungsgemäß hergestellte α-ÄDU-Monophosphat besitzt den überraschenden Vorteil, daß es virostatisch wirkt, ohne in die DNS eingebaut zu werden. Es stellt somit keine genetische Gefahr dar.

Die erfindungsgemäßen Verbindungen können lokal, oral oder parenteral in einem üblichen pharmazeutisch verträglichen Verdünnungsmittel oder Träger angewendet werden.

Anwendungsbeispiele

1. Erzeugung von Herpes-Virenstämmen, die gegenüber den Nukleosid-Analoga resistent sind.

Man züchtet Herpes-Viren in Gewebekulturen von menschlichen Fibroblastzellen über 1 bis 5 Passagen in Gegenwart von Nukleosiden, wie beispielsweise ÄDU (Äthyldesoxyuridin), Konzentration: 20 µg/ml, TFT (Trifluorthymidin) Konzentration 5 bis 10 µg/ml, ARA-A-MP (Arabinofuranosyladenin-Monophosphat), Konzentration 20 µg/ml, Acycloguanosin (ACG), Konzentration 5 bis 30 µg/ml , wobei der Saatvirus auf die Cornea der Tiere (Hamster, Kaninchen) durch Aufträufeln übertragen wird. Zwei Stunden nach der Übertragung erfolgt die Behandlung der Cornea lokal mit den entsprechenden Nukleosiden in den oben angegebenen Konzentrationen. Dabei zeigt sich, daß die Viren für die Nukleoside resistent werden. Die Bewertung der Infektion erfolgt durch Anfärben der Hornhaut mit Fluorescin-Na-Lösung (1 %) und anschließende Farbphotodokumentation.

2. Behandlung der resistenten Herpes-Infektionen mit den Monophosphaten der Erfindung:

Die Behandlung der Herpes-Keratitis bei Kaninchen, erzeugt mit den resistenten Stämmen, erfolgt lokal mit folgenden Wirkstoffen: JDU-MP (Joddesoxyuridin-Monophosphat), Konzentration 0,1 bis 1 %, ÄDU-MP (Äthyldesoxyuridin-Monophosphat), Konzentration: 0,1 bis 8 %, TFT-MP (Trifluorthymidin-Monophosphat), Konzentration: 0,1 bis 2 %,

ARA-A-MP (Arabinofuranosyladenin-Monophosphat), Konzentration: 0,2 bis 2 %, Acycloguanosin-Monophosphat, Konzentration: 0,5 bis 5 %, bis zu 7 mal täglich für die Dauer von bis zu 11 Tagen. Bei dieser Anwendungsweise läßt sich die durch resistente Herpesviren verursachte Infektion wirksam bekämpfen, während die Nukleoside bei entsprechender Anwendung und Konzentration unwirksam sind. Ähnlich kann die Behandlung der HSV-Infektionen durch resistente Viren auch durch parenterale Verabreichung der Monophosphate erfolgen.

## Patentansprüche

1. Nucleotide der allgemeinen Formel I:

$$\text{Base} - \text{R} - \text{O} - \overset{\overset{\displaystyle \text{O}}{\|}}{\underset{\underset{\displaystyle \text{OH}}{|}}{\text{P}}} - \text{OH} \qquad \text{(I)}$$

worin:

"Base" einen Purinbasenrest, Pyrimidinbasenrest, Pyridon-2-basen-rest oder 3,6-Dihydropyridon-2-basenrest bedeutet, wobei

(a) der Pyrimidinbasenrest und der Pyridon-2-basenrest in 4-Stellung eine Aminogruppe tragen können;

(b) der Pyridon-2-basenrest in 5-Stellung durch ein Halogen substituiert sein kann; und

(c) der Pyrimidinbasenrest in 5-Stellung durch Halogen, einen geradkettigen oder verzweigten $(C_2-C_6)$Alkylrest, $(C_2-C_6)$Alkenylrest, $(C_2-C_6)$Halogenalkylrest oder $(C_2-C_6)$Halogenalkenylrest substituiert sein kann, in 6-Stellung durch Chlor substituiert sein kann und, wenn in 5-Stellung ein $(C_2-C_6)$Alkylrest steht, in 3-Stellung durch einen geradkettigen oder verzweigten $(C_1-C_4)$Alkylrest substituiert sein kann;
und

R einen Pentofuranosylrest der allgemeinen Formel II darstellt:

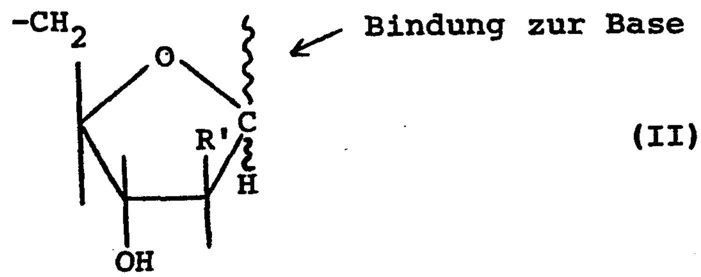

(II)

worin R' für ein Halogenatom, H oder OH steht; oder

R eine gerade oder verzweigte Alkylenkette mit 2 bis 6 Kohlenstoffatomen, die gegebenenfalls ein Äther-O-Atom in der Kette enthält, bedeutet,

wobei Halogen für F, Cl, Br, J steht;

sowie die Cyclophosphatester mit der OH-Gruppe in 3-Stellung des Pentofuranosylrests R.

2. Nucleotide nach Anspruch 1, dadurch gekennzeichnet, daß der Rest "Base" einen Purinbasenrest, Pyrimidinbasenrest oder Pyridon-2-basenrest bedeutet, wobei

(a) der Pyrimidinbasenrest und der Pyridon-2-basenrest in 4-Stellung eine Aminogruppe tragen können,

(b) der Pyridon-2-basenrest in 5-Stellung durch ein Fluor-, Chlor-, Brom- oder Jodatom substituiert sein kann; und

(c) der Pyrimidinbasenrest in 5-Stellung durch Halogen, einen geradkettigen oder verzweigten $(C_2-C_6)$Alkylrest, $(C_2-C_6)$Alkenylrest, $(C_2-C_6)$Halogenalkylrest oder $(C_2-C_6)$Halogenalkenylrest substituiert sein kann, wobei Halogen für Fluor, Chlor, Brom oder Jod steht; und

R einen Pentofuranosylrest der Formel II:

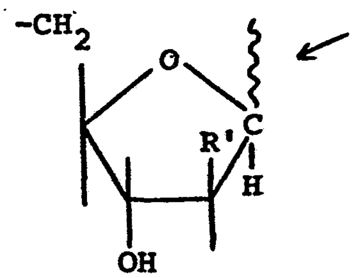

Bindung zur Base

(II)

worin R' für ein Fluor-, Chlor-, Brom- oder Jodatom steht; oder

eine gerade oder verzweigte Alkylenkette mit 2 bis 6 C-Atomen, die gegebenenfalls ein Äther-O-Atom in der Kette enhält; bedeutet.

3. Nucleotide nach Anspruch 1 oder 2, dadurch gekennzeichnet, daß die Purinbase das Purin selbst oder Adenin, Guanin, Hypoxanthin oder Xanthin ist und die Pyrimidinbase das Pyrimidin selbst oder Cytosin, Uracil, 5-Methylcytosin oder 5-Hydroxymethylcytosin ist.

4. Nucleotide nach den Ansprüchen 1 bis 3, dadurch gekennzeichnet, daß der Substituent in 5-Stellung der Pyrimidinbase ein Äthyl-, Propyl-, Butyl-, Pentyl-, Vinyl-, Allyl-, 2-Halovinyl- oder 3-Haloalkylrest, wobei Halo für F,Cl und Br steht, ist.

5. Nucleotide nach Anspruch 4, dadurch gekennzeichnet, daß die Pyrimidinbase Uracil ist und R für den Pentofuranosylrest steht.

6. Nucleotide nach einem der Ansprüche 1 bis 5, worin der Begriff "Base" für einen Uracilrest steht, der allgemeinen Formel III:

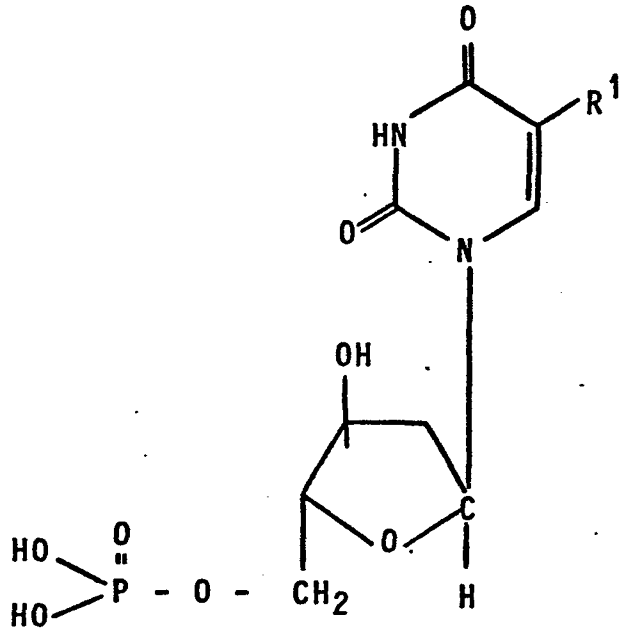

(III)

worin $R^1$ einen verzweigten oder geradkettigen $(C_2-C_6)$Alkylrest darstellt;

sowie der allgemeinen Formel IV:

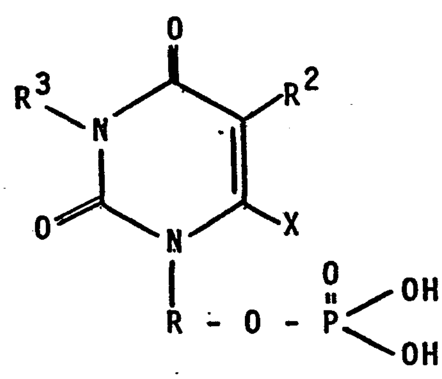

(IV)

worin

$R^2$ für einen geradkettigen oder verzweigten $(C_2-C_6)$Alkylrest steht und

$R^3$ für ein Wasserstoffatom oder einen geradkettigen oder verzweigten $(C_1-C_4)$Alkylrest steht,

X ein Wasserstoff- oder Chloratom bedeutet; und R die in Anspruch 1 genannten Bedeutungen hat.

7. Verfahren zur Herstellung der Verbindungen nach den Ansprüchen 1 bis 6, dadurch gekennzeichnet, daß man eine Verbindung der allgemeinen Formel V:

$$\text{Base} - R - OH \qquad (V)$$

worin die Reste Base und R die oben genannten Bedeutungen haben, entweder mit Phosphoroxychlorid in einem Lösungsmittel phosphoryliert und das erhaltene Phosphat der Formel I mit Äther, Petroläther oder einem Gemisch dieser Lösungsmittel aus dem Reaktionsgemisch ausfällt oder

ein Base-Metallsalz, insbesondere ein Base-Alkali- oder Quecksilbersalz, mit einer Verbindung der allgemeinen Formel (VI):

$$H_5C_6 - O - \underset{\underset{H_5C_6-O}{|}}{\overset{\overset{O}{\|}}{P}} - O - R - Cl \qquad (VI)$$

wobei "Base" und R die oben genannten Bedeutungen haben,

umsetzt und anschließend die Phenylgruppe am Phosphatrest durch Behandlung mit Phosphordiesterase entfernt.

8. Arzneimittel, enthaltend wenigstens eine Verbindung gemäß den Ansprüchen 1 bis 6 als Wirkstoff in einem üblichen pharmazeutischen Träger und/oder Verdünnungsmittel, gegebenenfalls in Verbindung mit Arzneimittelzusätzen.